# EUROPEAN PATENT APPLICATION

(11) **EP 4 435 797 A1**
(43) Date of publication of application: **25.09.2024**
(21) Application number: 23165558.0
(22) Date of filing: 30.03.2023
(51) Int. Cl.: G16H 20/70, G16H 50/30

(54) **METHOD TO CORRECT COGNITIVE TESTS FOR MULTI-DOMAIN EFFECTS**

(30) Priority: 22.03.2023 US 202363453779 P
(71) Applicant: Koninklijke Philips N.V., 5656 AG Eindhoven (NL)
(72) Inventor: KLAMING, Laura, Eindhoven (NL); HART DE RUIJTER-BEKKER, Evelijne Machteld, Eindhoven (NL); VAN EE, Raymond, 5656AG Eindhoven (NL)
(74) Representative: Philips Intellectual Property & Standards

(57) **Abstract**

The present invention relates to a computer-implemented method to correct a cognitive test of a patient for multi-domain effects. The method comprises the steps of acquiring a first test score of the patient obtained at a first cognitive test to be corrected for multi-domain effects, obtaining a core cognitive function of the patient measured by the first cognitive test, obtaining at least one second cognitive function of the patient measured by the first cognitive test, determining at least one second cognitive test measuring the at least one second cognitive function of the patient, and acquiring at least one second test score of the patient obtained at the at least one second cognitive test. The method comprises further the steps of scaling the first test score of the patient obtained at the first cognitive test and the at least one second test score of the patient obtained at the at least one second cognitive test to a common scale, performing a calculation with the scaled first test score of the first cognitive test and the scaled at least one second test score of the at least one second cognitive test thereby obtaining a calculation result, comparing the calculation result with a predetermined threshold thereby deriving a measure for an impairment of the patient in the core cognitive function measured by the first cognitive test, and providing the measure for an impairment of the patient in the core cognitive function measured by the first cognitive test.

## Description

### FIELD OF THE INVENTION

The present invention relates to a computer-implemented method to correct a cognitive test of a patient for multi-domain effects, a data processing apparatus, a computer program, and a computer-readable storage medium.

### BACKGROUND OF THE INVENTION

Cognitive tests typically measure multiple cognitive functions or domains. For instance, while aspects of executive function such as working memory are at the core of the Trail Making Test B (TMT B), the test also measures visual search processes and attention. Similarly, the Controlled Oral Word Association Test (COWAT) is a measure of verbal function, but it also measures aspects of executive function such as processing speed. Cognitive tests can be mapped onto the respective cognitive domains that are measured by the test, and factor loadings of the different cognitive domains for each cognitive test are tabulated in various publications.

A major reason why it can be problematic that a given cognitive test measures multiple cognitive functions or domains combined is that if a patient scores below the norm on a cognitive test, it can be difficult to determine based on the patient's performance on that specific test what cognitive function exactly is impaired. For instance, if the patient scores below the norm on the TMT B which measures working memory, visual search processes and attention, the neuropsychologist is not able to derive from this test result which of these cognitive functions or domains is impaired. Being able to separate cognitive functions measured with one cognitive test, and thus separating multi-domain effects of the cognitive test, can improve diagnosis. In the case of the TMT B, being able to separate working memory from visual search and attention enables the neuropsychologist to determine which of these cognitive functions is impaired. Usually, these multi-domain effects are not corrected. In the current way of working, this information is obtained by conducting multiple cognitive tests and by conducting task analysis afterwards. Without correcting for these multi-domain effects, a neuropsychologist will have to conduct several other tests that measure these cognitive functions, or ideally only one of them, in order to manually correct the outcome of the TMT B. Hence, being able to correct for multi-domain effects may also decrease the time needed for a neuropsychological assessment.

The inventors of the present invention have thus found that it would be advantageous to have a method for correcting cognitive tests for multi-domain effects that can decrease test time and lead to improved first-time right diagnosis.

### SUMMARY OF THE INVENTION

It is an object of the present invention to provide an improved method of correcting cognitive tests for multi-domain effects that can be integrated into a cognitive testing platform and can support a neuropsychologist in updating of a hypothesis and in test selection.

The object of the present invention is solved by the subject matter of the independent claims, wherein further embodiments are incorporated in the dependent claims.

The described embodiments similarly pertain to the computer-implemented method to correct a cognitive test of a patient for multi-domain effects, the data processing apparatus, the computer program, and the computer-readable storage medium. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a computer-implemented method to correct a cognitive test of a patient for multi-domain effects. The method comprises the steps of acquiring a first test score of the patient obtained at a first cognitive test to be corrected for multi-domain effects, obtaining a core cognitive function of the patient measured by the first cognitive test, obtaining at least one second cognitive function of the patient measured by the first cognitive test, determining at least one second cognitive test measuring the at least one second cognitive function of the patient, and acquiring at least one second test score of the patient obtained at the at least one second cognitive test. The method comprises further the steps of scaling the first test score of the patient obtained at the first cognitive test and the at least one second test score of the patient obtained at the at least one second cognitive test to a common scale, performing a calculation with the scaled first test score of the first cognitive test and the scaled at least one second test score of the at least one second cognitive test thereby obtaining a calculation result, comparing the calculation result with a predetermined threshold thereby deriving a measure for an impairment of the patient in the core cognitive function measured by the first cognitive test, and providing the measure for an impairment of the patient in the core cognitive function measured by the first cognitive test.

Thus, the proposed method to correct individual cognitive tests for multi-domain effects provides purer measures of cognitive functioning, and can be, for example, automated and implemented into a cognitive test platform. The proposed method can be used as a clinical decision support that when integrated into a neuropsychological assessment or cognitive test battery like a cognitive testing platform can support the neuropsychologist in finding or updating a hypothesis regarding a disablement of a patient and in test selection. Test selection can preferably be automated based on a performance of the patient in a previous cognitive test. The proposed method can decrease test time and lead to improved first-time right diagnosis.

The method is based on known factor loadings of cognitive tests onto cognitive domains, as cognitive tests typically measure several cognitive functions or multiple domains. The method can correct for multi-domain effects in real-time and offer real-time clinical decision support. By doing that, individual cognitive tests provide more information and neuropsychologists can update their hypothesis and test selection based on this input. With this correction of multi-domain effects, when a patient performs a cognitive test below the norm, it can be concluded which cognitive function or domain exactly is impaired, without manually conducting multiple cognitive tests and conducting task analysis afterwards.

According to the method of the present invention, a first test score of the patient is obtained at a first cognitive test that is to be corrected for multi-domain effects. The cognitive functions measured with this first test, as well as a core cognitive function of this first test is determined. Then, another test is selected from a data base that measures at least one cognitive function measured by the first test, which is not the core cognitive function. This another test is performed by the patient and the second test score is acquired. The first test score and the second test score are scaled to a common scale, if necessary. With these scaled test scores, a calculation can be performed that provides a calculation result that maintains a measure for the ability of the patient in the core cognitive function of the first test, while the other cognitive functions measured by the first test are filtered out or subtracted. By comparing the calculation result with a threshold or at least comparative tabulated data, a measure for an impairment of the patient in the core cognitive function measured by the first cognitive test can be derived and provided to a neuropsychologist.

For example, with respect to the TMT B, a measure of the patient's working memory without influences of visual search processes and attention can be derived with this method. For each individual cognitive test, e.g. in a test battery, it can be determined what the core cognitive domain of the test is, e.g. based on factor loadings. In the case of the TMT B, working memory would be the core cognitive domain. Then, it can be determined based on existing scientific research, like known factor analysis and task analysis from the literature on healthy people, or based on new analyses, which cognitive domains are involved in performing the respective test and which other tests from the test battery measure these cognitive functions. In the case of the TMT B, the test also measures visual search processes and attention. Other tests that also measure these functions are the TMT A that measures visual search processes and the O Search test that measures attention. Subsequently, performance on all cognitive tests in the test battery can be scaled, e.g. to a scale from 0 to 100. After that, the scaled test scores of the at least one second test can be subtracted from the scaled test score of the original cognitive test, the first test. In the case of the TMT B, the scaled TMT A performance and the scaled O Search test performance are be subtracted from the scaled TMT B performance. This calculation result provides a measure for a possible impairment of the patient in the core cognitive function. Although subtraction is used as a straightforward example to correct for multi-domain effects in this example, the inventors realize that the correction method according to the invention should not be limited to this specific example of the subtraction. Other calculation methods for the correction are also possible and can easily be embedded in this approach. Conventional methods from mathematics frequently use dividing by (the sum of) the individual test result. This could then lead to a ratio or a percentage score. Another more general method might include a calculation of the weighted arithmetic mean.

In an embodiment of the invention, a pattern analysis can be conducted over the obtained scores of the respective tests that are conducted during the method according to the invention, which are, for the example of the TMT B: TMT B, TMT A, and the O Search Test. If for instance an effect is found on the TMT B, but not on the TMT A, the patient likely has executive impairment. If an effect is found on both, the patient likely also has a visual search impairment.

In a further embodiment, a similar method may be developed for patients with functional or structural, i.e. anatomical, deficiencies. For this embodiment, the output of medical scans may be used. Information from Magnetic Resonance Imaging (MRI) and/or functional Magnetic Resonance Image (FMRI) can be used to refine the proposed correction method. For example, patients who have suffered from brain damage caused by a stroke in the parietal cortex exhibit characteristic anatomical and functional deficiency signatures, similar among this specific group of patients. These specific stroke patients may not be able to direct visual attention to a focused area because for directing visual attention the parietal cortex is key. If it is known from the medical scan that a stroke patient misses out on a significant number or area of neurons and/or functionality within the parietal cortex, it is then known that attention will not be involved as a secondary domain. This evaluation can be done by a medical doctor or automatically based upon lookup tables, similar to the method described above. In this example, the parietal cortex and stroke are specified, but this approach can be generalized to any brain part that can be responsible for cognitive performance during a primary or secondary task. In addition, in the above example is explained with respect to magnetic resonance imaging, but any brain scan method may be used. In a related embodiment also information from temporal processing brain scans (EEG) can be used.

In an embodiment of the invention, the calculation is a subtraction of the scaled at least one second test score from the scaled first test score.

In an embodiment of the invention, the predetermined threshold is 0.

For the subtraction method, a positive corrected score indicates no major impairment on the core cognitive function, while a negative corrected score can indicate impairment on the core cognitive function. Moreover, for a positive score, the higher the score, the larger the impairment on the cognitive function that is not the core cognitive function measured with the test. For a negative score, the lower the score, the larger the impairment on the core cognitive function.

In an embodiment of the invention, the calculation is a division of the scaled first test score by the scaled at least one second test score.

In an embodiment of the invention, the predetermined threshold is 1.

For the division method, a corrected score that is greater than 1 may indicate no impairment on the core cognitive function, while a score smaller than 1 indicates an impairment on the core cognitive function. For both methods, the corrected score on the TMT B should be used to compare a patient to a norm table. The norm might have to be established for this special use case.

In an embodiment of the invention, the first cognitive test measures a plurality of second cognitive functions. Thus, it may be possible that the respective first test is sensitive to a core cognitive function and in addition to at least one further cognitive function.

In an embodiment of the invention, the calculation is performed for each of the plurality of second cognitive functions. In this case, it might be necessary to acquire a test score for each of the further, i.e. second, cognitive functions. After scaling, these scaled test scores can be separately subtracted from the scaled test score of the first cognitive test, thereby each providing an indication for a cognitive impairment of the patient.

In an embodiment of the invention, the core cognitive function of the first cognitive test and/or the at least one second cognitive function of the first cognitive test are obtained from a data base containing factor loadings of each cognitive test in the data base with respect to a plurality of cognitive functions. Thus, a data base of a test battery can provide the respective data and factor loadings of the respective tests. In addition, this data base can contain a list of tests that measure the at least one second cognitive function such that an appropriate second test to be performed can be determined.

In an embodiment of the invention, the at least one second cognitive function of the first cognitive test is a core cognitive function of the at least one second cognitive test. Thus, the second test may be especially sensitive to the second cognitive function of the first test. However, it might also be possible to select a second test that measures the second cognitive function of the first test as a further cognitive function.

In an embodiment of the invention, the first test score and the at least one second test score are acquired by a digital neuropsychological test battery or a cognitive assessment platform. Thus, the described method can be automated in a digital neuropsychological test battery. The method will allow to better separate impairment in different cognitive domains during a neuropsychological assessment. This in turn will enable more accurate, first-time-right diagnosis and more effective treatment.

In an embodiment of the invention, the measure for the impairment of the patient in the core cognitive function measured by the first cognitive test is derived from the comparison of the calculation result with the predetermined threshold with respect to a norm table. Thus, tabulated values can be utilized in order to determine if and to which degree a patient is impaired in the respective cognitive domain.

In an embodiment of the invention, an impairment of the patient in the core cognitive function measured by the first cognitive test is determined in case the calculation result is smaller than the predetermined threshold. Thus, for example, if the calculation result is negative, i.e. smaller than a threshold of 0, this is an indication for the patient being impaired in the core cognitive function. Alternatively, this result at least may indicate that an impairment in the core cognitive function may be more severe than an impairment in the second cognitive function of the first test.

According to another aspect of the invention, there is provided a data processing apparatus comprising a processor configured to perform the steps of the method according to any of the preceding embodiments. This data processing apparatus may be part of an automated digital neuropsychological test battery.

According to another aspect of the invention, there is provided a computer program comprising instructions which, when the program is executed by a computer, cause the computer to carry out the steps of the method according to any of the preceding embodiments. The computer program element can be performed on one or more processing units, which are instructed to perform the method to correct a cognitive test of a patient for multi-domain effects.

According to another aspect of the invention, there is provided a computer-readable storage medium comprising instructions which, when executed by a computer, cause the computer to carry out the steps of the method according to any of the preceding embodiments.

Thus, the benefits provided by any of the above aspects equally apply to all of the other aspects and vice versa.

In summary, the invention relates to a computer-implemented method to correct a cognitive test of a patient for multi-domain effects. The method comprises the steps of acquiring a first test score of the patient obtained at a first cognitive test to be corrected for multi-domain effects, obtaining a core cognitive function of the patient measured by the first cognitive test, obtaining at least one second cognitive function of the patient measured by the first cognitive test, determining at least one second cognitive test measuring the at least one second cognitive function of the patient, and acquiring at least one second test score of the patient obtained at the at least one second cognitive test. The method comprises further the steps of scaling the first test score of the patient obtained at the first cognitive test and the at least one second test score of the patient obtained at the at least one second cognitive test to a common scale, performing a calculation with the scaled first test score of the first cognitive test and the scaled at least one second test score of the at least one second cognitive test thereby obtaining a calculation result, comparing the calculation result with a predetermined threshold thereby deriving a measure for an impairment of the patient in the core cognitive function measured by the first cognitive test, and providing the measure for an impairment of the patient in the core cognitive function measured by the first cognitive test.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows block a diagram of the method to correct a cognitive test of a patient for multi-domain effects according to an embodiment of the invention.
Fig. 2 shows block diagram of an exemplary method to correct a cognitive test of a patient for multi-domain effects of the TMT B test according to an embodiment of the invention.
Fig. 3 shows an example of a method according to an embodiment of the invention with subtraction and division correction methods for a patient without impairment in the core cognitive function (Patient A) and with impairment in the core cognitive function (Patient B).
Fig. 4 shows a schematic setup of a data processing apparatus according to an embodiment of the invention.
Fig. 5 shows block a diagram of a method to correct a cognitive test of a patient for multi-domain effects according to an embodiment of the invention.

### DETAILED DESCRIPTION OF EMBODIMENTS

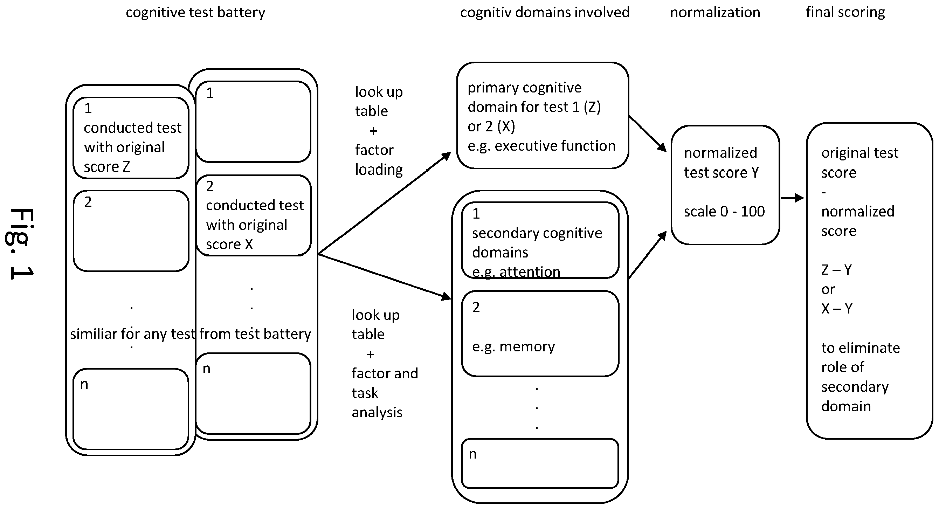

Fig. 1 shows block a diagram of the method to correct a cognitive test of a patient 110 for multi-domain effects according to an embodiment of the invention. This method corrects individual cognitive tests for multi-domain effects in order to get purer measures of cognitive functioning, in the example of the TMT B in order to get a measure of working memory without visual search processes and attention. For each individual cognitive test (e.g. in the ISC test battery), it will be determined what its core cognitive domain is (e.g. based on factor loadings). In the case of the TMT B, working memory would be the core cognitive domain. Then, it will be determined based on existing scientific research (e.g. known factor analysis, and task analysis from the literature on healthy people, or from additional analyses) which cognitive domains are involved in performing the given test and which other tests measure these cognitive functions. In the case of the TMT B, the test also measures visual search processes and attention. Other tests that also measure these functions are, for example, the TMT A (visual search processes) and the O Search test (attention). Subsequently, performance on all cognitive tests in the test battery will be scaled (e.g. to a 0-100 scale). After that, the scaled test score will be subtracted from the original cognitive test score. In the case of the TMT B, TMT A performance (scaled) and O Search test performance (scaled) will be subtracted from TMT B performance (scaled).

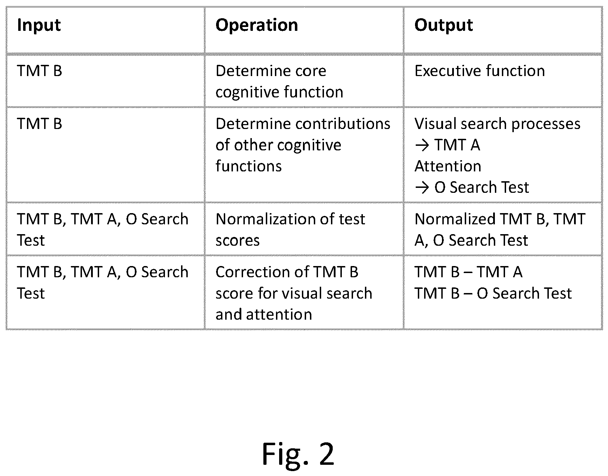

Fig. 2 shows block diagram of an exemplary method to correct a cognitive test of a patient 110 for multi-domain effects of the TMT B test according to an embodiment of the invention. For the TMT B, as core cognitive function the executive functions are determined. After that, the contributions of other cognitive functions to the TMT B are determined, which are in this example of the TMT B the visual search processes that can for instance be measured with the TMT A test and attention that can for instance be measured with the O Search test. For all included tests, the test scores are scaled to a scale from 0 to 100, for example. Then, the test score of the TMT B is corrected for visual search and attention by subtraction of the respective scaled test scores.

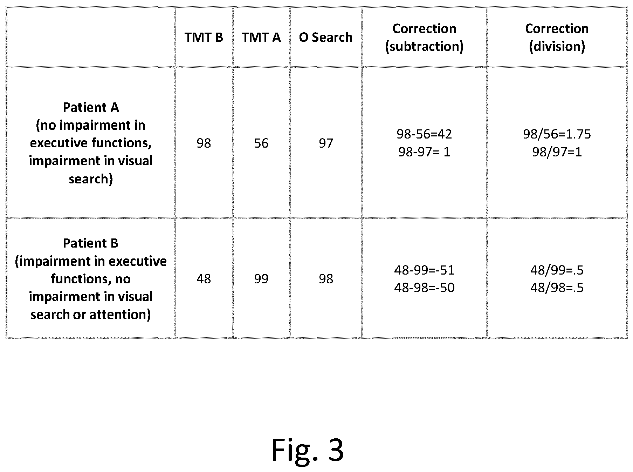

Fig. 3 shows an example of a method according to an embodiment of the invention with subtraction and division correction methods for a patient 110 without impairment in the core cognitive function (Patient A) and for a patient 110 with impairment in the core cognitive function (Patient B). This figure provides an example with scores on each of the tests, i.e. the TMT B, TMT A, and O Search test to better illustrate how the subtraction and division methods work. For instance, if a patient does not have an impairment in executive functions or attention, but does have an impairment in visual search (Patient A), he would score relatively high on the TMT B (e.g. 98) and relatively low on the TMT A (e.g. 56) and relatively high on the O Search Test (e.g. 97). Using the subtraction correction method, this would result in a corrected TMT B score of 98-56 = 42 for visual search and in a corrected TMT B score of 98-97 = 1 for attention, clearly indicating an impairment on TMT A. Using the division correction method, this would result in a corrected TMT B score of 98/56 = 1.75 for visual search and in a corrected TMT B score of 98/97 = 1 for attention, clearly indicating no impairment on TMT B. To give another example, if a patient has impairment in executive functions, but not in visual search or attention, he would score relatively low on the TMT B (e.g. 48) and relatively high (or within the norm) on the TMT A (e.g. 99) and the O Search Test (e.g. 98). Using the division correction method, this would result in a corrected TMT B score of 48-99= -51 for visual search and in a corrected TMT B score of 48-98 = -50 for attention, clearly indicating an impairment on the core cognitive function. Using the division correction method, this would result in a corrected TMT B score of 48/99 = 0.5 for visual search and in a corrected TMT B score of 48/98 = 0.5 for attention, clearly indicating an impairment on the core cognitive function.

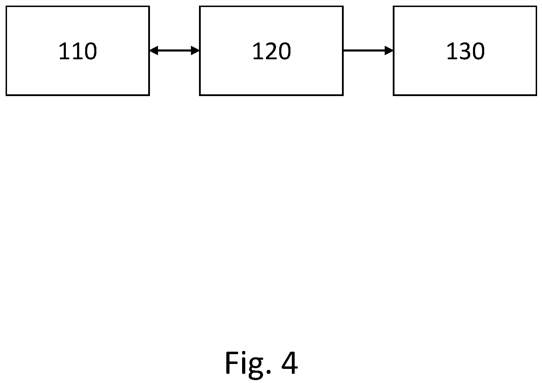

Fig. 4 shows a schematic setup of a data processing apparatus 120 according to an embodiment of the invention. The data processing apparatus 120 is in communication with the patient 110 and acquires the respective test scores of the patient 110 on the respective tests. After performing the method according to an embodiment of the invention, the measure of an impairment 130 of the patient 110 is provided by the data processing apparatus 120.

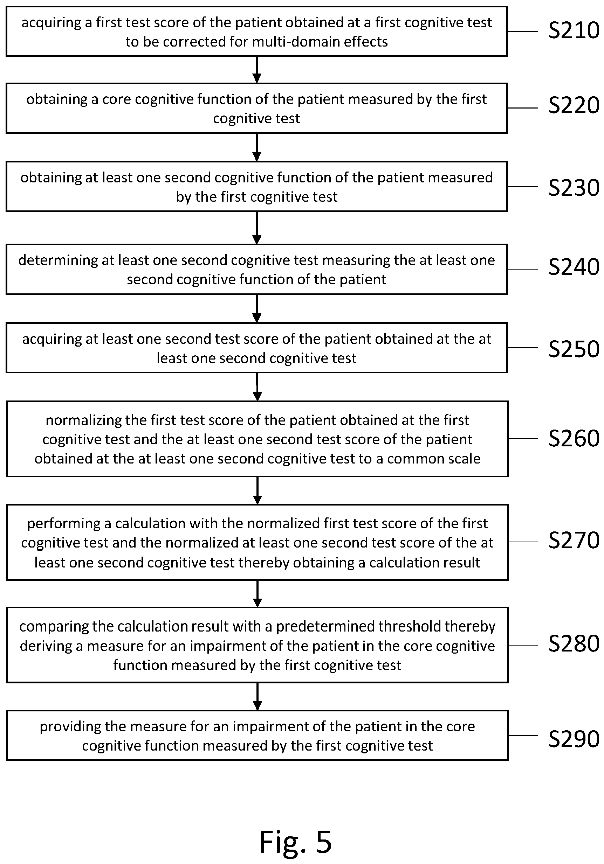

Fig. 5 shows block a diagram of a method to correct a cognitive test of a patient 110 for multi-domain effects according to an embodiment of the invention. The method comprises the steps of acquiring a first test score of the patient obtained at a first cognitive test to be corrected for multi-domain effects S210, obtaining a core cognitive function of the patient measured by the first cognitive test S220, obtaining at least one second cognitive function of the patient measured by the first cognitive test S230, determining at least one second cognitive test measuring the at least one second cognitive function of the patient S240, and acquiring at least one second test score of the patient obtained at the at least one second cognitive test S250. The method comprises further the steps of normalizing the first test score of the patient obtained at the first cognitive test and the at least one second test score of the patient obtained at the at least one second cognitive test to a common scale S260, performing a calculation with the scaled first test score of the first cognitive test and the scaled at least one second test score of the at least one second cognitive test thereby obtaining a calculation result S270, comparing the calculation result with a predetermined threshold thereby deriving a measure for an impairment of the patient in the core cognitive function measured by the first cognitive test S280, and providing the measure for an impairment of the patient in the core cognitive function measured by the first cognitive test S290.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 110: patient
- 120: data processing apparatus
- 130: measure for an impairment

## Claims

1. A computer-implemented method to correct a cognitive test of a patient (110) for multi-domain effects, the method comprising the steps of:
acquiring a first test score of the patient (110) obtained at a first cognitive test to be corrected for multi-domain effects S(210);
obtaining a core cognitive function of the patient measured by the first cognitive test (S220);
obtaining at least one second cognitive function of the patient measured by the first cognitive test (S230);
determining at least one second cognitive test measuring the at least one second cognitive function of the patient (S240);
acquiring at least one second test score of the patient (110) obtained at the at least one second cognitive test (S250);
scaling the first test score of the patient obtained at the first cognitive test and the at least one second test score of the patient obtained at the at least one second cognitive test to a common scale (S260);
performing a calculation with the scaled first test score of the first cognitive test and the scaled at least one second test score of the at least one second cognitive test thereby obtaining a calculation result (S270);
comparing the calculation result with a predetermined threshold thereby deriving a measure for an impairment of the patient in the core cognitive function measured by the first cognitive test (S280); and
providing the measure for an impairment (130) of the patient (110) in the core cognitive function measured by the first cognitive test (S290).

2. The method according to claim 1, wherein the calculation is a subtraction of the scaled at least one second test score from the scaled first test score.

3. The method according to claim 2, wherein the predetermined threshold is 0.

4. The method according to claim 1, wherein the calculation is a division of the scaled first test score by the scaled at least one second test score.

5. The method according to claim 4, wherein the predetermined threshold is 1.

6. The method according to any of the preceding claims, wherein the first cognitive test measures a plurality of second cognitive functions.

7. The method according to claim 6, wherein the calculation is performed for each of the plurality of second cognitive functions.

8. The method according to any of the preceding claims, wherein the core cognitive function of the first cognitive test and/or the at least one second cognitive function of the first cognitive test are obtained from a data base containing factor loadings of each cognitive test in the data base with respect to a plurality of cognitive functions.

9. The method according to any of the preceding claims, wherein the at least one second cognitive function of the first cognitive test is a core cognitive function of the at least one second cognitive test.

10. The method according to any of the preceding claims, wherein the first test score and the at least one second test score are acquired by a digital neuropsychological test battery or a cognitive assessment platform.

11. The method according to any of the preceding claims, wherein the measure for the impairment of the patient in the core cognitive function measured by the first cognitive test is derived from the comparison of the calculation result with the predetermined threshold with respect to a norm table.

12. The method according to any of the preceding claims, wherein an impairment of the patient in the core cognitive function measured by the first cognitive test is determined in case the calculation result is smaller than the predetermined threshold.

13. A data processing apparatus (120) comprising a processor configured to perform the steps of the method according to any of claims 1 to 12.

14. A computer program comprising instructions which, when the program is executed by a computer, cause the computer to carry out the steps of the method according to any of claims 1 to 12.

15. A computer-readable storage medium comprising instructions which, when executed by a computer, cause the computer to carry out the steps of the method according to any of claims 1 to 12.
